# EUROPÄISCHE PATENTANMELDUNG

(11) **EP 1 550 436 A1**
(43) Veröffentlichungstag der Anmeldung: **06.07.2005**
(21) Anmeldenummer: 04105823.1
(22) Anmeldetag: 17.11.2004
(51) Int. Cl.: A61K 7/48, A61K 7/50, A47K 7/02, A61B 17/54

(54) **Kosmetikartikel mit verbesserter Penetration eines Wirkstoffs durch gleichzeitig anzuwendende abrasive Vorrichtung**

(30) Priorität: 26.11.2003 DE 10355229
(71) Anmelder: Beiersdorf AG, 20245 Hamburg (DE)
(72) Erfinder: Staeb, Franz, 21379 Echem (DE); Blatt, Thomas, 21379 Wedel (DE); Schmidt, Melanie, 21031 Hamburg (DE); Lenz, Holger, 22049 Hamburg (DE)

(57) **Zusammenfassung**

Kosmetisches und/oder dermatologisches Produkt umfassend eine Vorrichtung zum Abtragen von Haut und eine wirkstoffhaltige kosmetische und/oder dermatologische Zubereitung dadurch gekennzeichnet, dass bei der Anwendung des Produktes gleichzeitig die Vorrichtung angewendet und die Zubereitung dosiert wird.

## Beschreibung

Die vorliegende Erfindung betrifft Kosmetikartikel mit verbesserter Penetration eines Wirkstoffs durch gleichzeitig anzuwendende abrasive Vorrichtung.

Degenerative Hauterscheinungen im Sinne der vorliegenden Schrift sind Falten, Erschlaffung, Degeneration, Altersflecken, Pigmentstörungen, Durchblutungsstörungen, Juckreiz, Stressempfindlichkeit, trockene Haut, Cellulite, Dermoverspannungen, Ermüdungszustände.

Vorrichtungen zum Abtragen von Haut im Sinne der vorliegenden Schrift sind Schwämme, mit abrasiven Partikeln besetzte, gewebte oder nichtgewebte Textilien, abrasive Fasern oder Partikel enthaltende Textilien wie Waschhandschuhe mit oder ohne Kokos oder Hanffasern, Seegurkenskellett (Luffa), grobgewebte Textilien.

Abrasive Partikel im Sinne der vorliegenden Schrift sind beispielsweise Quarzsand, Mandelkleie, Polyethylen, Aluminiumtrioxid, gemahlene Fruchtkerne (Wallnuss- oder Aprikosenschalen), Salzkristalle, Jojoba-Blättchen, gemahlene Sepia-Schalen, Bims, Zeolithe, Siliciumdioxid.

Schwämme im Sinne der vorliegenden Schrift sind natürlich oder künstlich. Natürliche Schwämme sind beispielsweise Korallenschwämme oder Bimsschwämme, künstliche Schwämme sind beispielsweise grobporige Viskoseschwämme, Veloursschwämme, Polyester- oder Polyetherschwämme. Sie weisen eine zumeist eine unregelmäßige Zellstruktur und Poren unterschiedlicher Größe auf. Sie sind flexibel und elastisch.

Textilien im Sinne der vorliegenden Schrift sind Tücher, Fließe, Filz, Gewirke, Gewebe, Watten, aber auch Leinen, Schnüre, Seile und Zwirne.

Abrasive Fasern im Sinne der vorliegenden Schrift sind Natur- oder Kunstfasern, die durch ihre Oberflächenbeschaffenheit wie Härte oder Struktur oder durch ihre Abmessungen abrasive Eigenschaften aufweisen, wie beispielsweise Hanf-, Sisal-, Kokosfasern.

"Gleichzeitig" im Sinne der vorliegenden Schrift bedeutet auch mit geringem Zeitverzug. Insbesondere bedeutet "gleichzeitig anwenden" beispielsweise zweier Funktionen oder Eigenschaften eines beschriebenen Produktes, dass diese während eines Anwendungsvorgangs eines Produktes beide zum Tragen kommen. Dies kann entweder dadurch erreicht werden, dass aufeinander folgend beide Funktionen oder Eigenschaften zum Tragen kommen, etwa indem sie vor der Anwendung physisch trennbar oder getrennt vorliegen. In diesem Fall umfasst gleichzeitig einen Zeitraum von 0 bis 30 Sekunden. Bevorzugt kommen Funktionen oder Eigenschaften gleichzeitig zum Tragen, indem sie in einem Artikel integriert angeordnet sind. Auch in diesem Fall umfasst gleichzeitig ein Zeitinterval von 0 bis etwa 5 Sekunden, wenn beispielsweise die Orte der Funktionen oder Eigenschaften voneinander entfernt angeordnet sind, beispielsweise 20 mm, und bei der Anwendung mit einer gewissen Geschwindigkeit, beispielsweise 50 mm/s entlang eines Anwendungsweges aktiviert werden.

Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Die Schrift WO 94/18879 offenbart Vorrichtungen zur kosmetischen Schälung der Haut mit einem näher bestimmten textilen Flächengebilde.

Die Schriften US 4459987 und US 4712552 offenbaren biegsame kosmetische Schmirgelbögen auf Basis bestimmter Polymere, die mindestens einseitig Schmirgelpartikel tragen.

Die Schrift EP 1219254 offenbart einen Apparat zum Abtragen von Haut.

Über ein bloßes Schälen der Haut hinausgehende Anwendungen solcher Vorrichtungen wird hingegen in keinem der Dokumente etwas offenbart.

Es hat sich für den Fachmann nicht vorhersehbar herausgestellt, dass ein kosmetisches und/oder dermatologisches Produkt umfassend eine Vorrichtung zum Abtragen von Haut und eine wirkstoffhaltige kosmetische und/oder dermatologische Zubereitung, wobei bei der Anwendung des Produktes gleichzeitig die Vorrichtung angewendet und die Zubereitung dosiert wird, den Mängeln des Standes der Technik abhelfen. Bei Anwendung eines solchen Produktes wirkt der mechanische Reiz als physiologischer Stimulus der Haut, der durch gleichzeitige Anwendung des Wirkstoffs verstärkt oder ausgeglichen wird.

Es wurde weiter gefunden, dass es bevorzugt ist, wenn die Vorrichtung ein grobporiger Gesichtshandschuh ist. Durch dessen Anwendung wird die Haut zunächst von Verschmutzungen gereinigt. Der oben beschriebene Effekt tritt noch stärker hervor.

Besonders bevorzugt ist es, wenn die Zubereitung als W/O- Creme vorliegt. Dabei ist besonders bevorzugt, wenn in der Zubereitung ein Wirkstoff enthalten ist, der ganz besonders bevorzugt aus der Gruppe Antioxidantien gewählt wird. Ganz außergewöhnlich bevorzugt ist es, wenn er Ubichinon, Ubichinol, Kreatin und/oder Kreatinin darstellt.

Bei all dem ist es bevorzugt, wenn die Dosierung der Zubereitung durch die Vorrichtung bei ihrer Anwendung erfolgt. Die Erfindung umfasst auch die Verwendung einer solchen Vorrichtung zur Erhöhung der Penetration eines innerhalb von 30 s angewendeten kosmetischen und/oder dermatologischen Wirkstoffes, besonders bevorzugt zur Verbesserung des Energiestoffwechsels und/oder zur Anregung der Kollagenneogenese oder weiterer dermaler Komponenten. Darüber hinaus umfasst die Erfindung auch ein nicht therapeutisches Verfahren zur Behandlung und Prophylaxe degenerativer Hauterscheinungen durch Anwenden eines oben beschriebenen Produktes.

Bevorzugt enthält die als Kosmetikartikel anzuwendende, abrasive Vorrichtung gleichzeitig, im Sinne der Erfindung, Wirkstoffe, deren Einsatzkonzentration im Bereich von 0,0001% bis 50 Gewichts%, besonders bevorzugt 0,01% bis 10 Gewichts% liegt (jeweils bezogen auf das Gesamtgewicht der Zubereitung).

Bei Anwendung der erfindungsgemäß verwendeten Vorrichtung gleichzeitig mit einer kosmetischen oder topischen dermatologischen Zubereitung ist in überraschender Weise eine wirksame Behandlung, aber auch eine Prophylaxe
- von bestimmten degenerativen Erscheinungen der Haut (z. B. Hauterschlaffung, Falten, Tränensäcke, Altersflecken, Teleangiektasien, Störung der Osmolytbalance, des natürlichen Haut pH-Wertes sowie Schwund der epidermalen und dermalen Zellschichten, der Bestandteile des Bindegewebes, der Retezapfen und Kapillargefässe der Haut) und/oder der Hautanhangsgebilde,
- von umweltbedingten (durch Rauchen, Smog, reaktive Sauerstoffspecies, freie Radikale und dergleichen verursachten) negativen Veränderungen der Haut und der Hautanhangsgebilde,
- von defizitären, sensitiven oder hypoaktiven Hautzuständen oder defizitären, sensitiven oder hypoaktiven Zustände von Hautanhangsgebilden,
- bei verringerter Hautdicke, Dermoverspannungen
- bei Hauterschlaffung, Hautermüdung, Schwangerschaftsstreifen
- von Pigmentierungsstörungen,
- von Hornschichtbarrierestörungen,
- bei Veränderungen des transepidermalen Wasserverlustes und des normalen Feuchtigkeitsgehaltes der Haut,
- bei Veränderung des Ceramid-, Lipid- und Energiestoffwechsels der gesunden Haut,
- von Haarausfall und für verbessertes Haarwachstum,
- bei Abweichungen von der normalen Zell-Zell-Kommunikation in der Haut,
- bei Veränderungen der normalen Fibroblasten- und Keratinozytenproliferation,
- bei Veränderungen der normalen Fibroblasten- und Keratinozytendifferenzierung,
- von entzündlichen Hautzuständen sowie atopischem Ekzem, seborrhoischem Ekzem, polymorpher Lichtdermatose, Psoriasis, Vitiligo
möglich.

Der erfindungsgemäße Vorrichtung samt Wirkstoff wirken aber auch in überraschender Weise
- zur Beruhigung von empfindlicher oder gereizter Haut,
- zur Aufrechterhaltung der normalen Kollagen-, Hyaluronsäure-, Elastin- und Glycosaminoglycan-Homeostase,
- zur Stimulation der Östrogen-Synthetase,
- bei gesteigerter Aktivierung proteolytischer Enzyme in der Haut, wie z. B. Metalloproteinasen,
- zur Stimulation der intrazellulären DNA-Synthese, insbesondere bei defizitären oder hypoaktiven Hautzuständen,
- bei Wundheilungsstörungen,
- zur Steigerung der Zellerneuerung und Regeneration der Haut,
- zur Verminderung der Produktion von Sebum,
- zur Verhinderung der Bildung und zur Entfernung von Comedonen und/oder zur Einstellung einer normalen Sebumhomeostase,
- zur Verhinderung oder Beseitigung von seborrhoischen Erscheinungen, ins-
- zur Steigerung der hauteigenen Schutz- und Reparaturmechanismen (beispielsweise für dysfunktionelle Enzyme, DNA, Lipide, Proteine),
- zur Vor- und Nachbehandlung bei topischer Anwendung von Laser- und Abschleifbehandlungen, die z. B. der Reduzierung von Hautfalten und Narben dienen, um den resultierenden Hautreizungen entgegenzuwirken und die Regenerationsprozesse in der verletzten Haut zu fördern.

Das Weglassen eines einzelnen Bestandteiles beeinträchtigt die einzigartigen Eigenschaften der Gesamtzusammensetzung. Daher sind alle angegebenen Bestandteile der erfindungsgemäßen Zubereitungen zwangsläufig erforderlich, um die Erfindung auszuführen.

Als grobporiger Gesichtshandschuh kann ein Sisalhandschuh, ein Peelinghandschuh aus Luffaschwamm oder ein mit Noppen besetzte Bürstenvorrichtungen in Handschuhform oder andere vergleichbare Vorrichtungen verwendet werden.

Vorteilhaft werden Antioxidantien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D,L-Carnosin, D-Carnosin, L-Carnosin und deren Derivate (z. B. Anserin), Carotinoide, Carotine (z. B. α-Carotin, β-Carotin, Ψ-Lycopin) und deren Derivate, Chlorogensäure und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glykosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Isoflavonoide, Licochalcone, Licochalcone A (insbesondere von *Radix Glycyrrhizae inflatae*) Folsäure und deren Derivate, Ubichinon und Ubichinol und deren Derivate, Vitamin C und Derivate (z. B. Ascorbylpalmitat, Mg-Ascorbylphosphat, Ascorbylacetat), Tocopherole und Derivate (z. B. Vitamin-E-acetat), Vitamin A und Derivate (Vitamin-A-palmitat) sowie Koniferylbenzoat des Benzoeharzes, Rutinsäure und deren Derivate, α-Glykosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z. B. ZnO, ZnSO₄), Selen und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den erfindungsgemäßen Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Erfindungsgemäße Zubereitungen können über die genannten Inhaltstoffe hinaus Öle, Wache, Lipide, Emulgatoren, Verdicker, Hautbefeuchtungsmittel, UV-Filter, Komplexbildner, Konservierungsstoffe, Parfüm, Puderrohstoffe, Pigmente , Farbstoffe enthalten.

Es ist bei all diesem im Einzelfalle möglich, dass die vorgenannten Konzentrationsangaben leicht über- oder unterschritten werden und dennoch erfindungsgemäße Zubereitungen erhalten werden. Dies kommt angesichts der breit streuenden Vielfalt an geeigneten Komponenten derartiger Zubereitungen für den Fachmann nicht unerwartet, so dass er weiß, dass bei solchen Über- oder Unterschreitungen der Boden der vorliegenden Erfindung nicht verlassen wird.

Die nachfolgenden Beispiele sollen die vorliegende Erfin dung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele

**Als Kokosseil-Handschuh wird ein handelsübliches Produkt z.B von Acca Kappa verwendet. Ein Luffaschwamm kann auch als Massageband und als** grobporiger Abschminkpad verwendet werden (z.B. von Wellsana)..

Bei der Anwendung wird die zu behandelnde Hautfläche mit dem sauberen, trockenen oder leicht angefeuchetem Schwamm einige Male, zum Beispiel 3 x mit leichtem Druck, beispielsweise mit kreisenden Bewegungen gerieben, ähnlich wie beid der Körpereinigung mit einem Waschlappen. Anschließend wird die Hautfläche mit der Zubereitung eingerieben. Dies erfolgt wie die normale Anwendung einer Creme oder Lotion.

### Beispiel 1

### Auftragen einer Gelcreme nach Behandlung der Haut mit einem Luffaschwamm:

| | Massengehalt (%) |
|---|---|
| Acrylates/C10-30 Al kyl Acrylate | 0.40 |
| Crosspolymer | |
| Carbomer | 0.20 |
| Xanthan Gum | 0.10 |
| Cetearyl Alkohol | 3.00 |
| C12-15 Alkyl Benzoate | 4.00 |
| Caprylic/Capric Triglyceride | 3.00 |
| Cyclometicone | 5.00 |
| Dimeticone | 1.00 |
| Kreatin | 0.20 |
| Glycerin | 3.00 |
| Natriumhydroxid | q.s. |
| Konservierung | q.s. |
| Parfum | q.s. |
| Wasser,demineralisiert | ad 100.0 |
| pH-Wert eingestellt auf 6.0 | |

### Beispiel 2

**Auftragen einer** W/O-Creme **nach Behandlung der Haut mit einem** grobporigem Abschminkpad:

| | Massengehalt (%) |
|---|---|
| Lameform TGI | 3.50 |
| Glycerin | 3.00 |
| Dehymuls PGPH | 3.50 |
| Ubichinon | 0.03 |
| Konservierungsmittel | q.s. |
| Parfum | q.s. |
| Wasser, demin. | ad 100.0 |
| Magnesiumsulfat | 0.6 |
| Isopropyl Stearate | 2.0 |
| Caprylyl Ether | 8.0 |
| Cetearyl Isononanoate | 6.0 |

### Beispiel 3

### Auftragen einer W/O/W-Creme nach Behandlung der Haut mit einem Kokosseil-Handschuh:

| | Massengehalt (%) |
|---|---|
| Glyceryl Stearate | 3.00 |
| PEG-100 Stearate | 0.75 |
| Behenylalkohol | 2.00 |
| Caprylic/Capric Triglyceride | 8.0 |
| Octyldodecanol | 5.00 |
| C12-15 Alkyl Benzoate | 3.00 |
| Taurin | 1,00 |
| Magnesium Sulfat (MgSO₄) | 0.80 |
| EDTA | 0.10 |
| Konservierung | q.s. |
| Parfum | q.s. |
| Wasser,demineralisiert | ad 100.0 |
| pH-Wert eingestellt auf 6.0 | |

## Patentansprüche

1. Kosmetisches und/oder dermatologisches Produkt umfassend eine Vorrichtung zum Abtragen von Haut und eine wirkstoffhaltige kosmetische und/oder dermatologische Zubereitung **dadurch gekennzeichnet, dass** bei der Anwendung des Produktes gleichzeitig die Vorrichtung angewendet und die Zubereitung dosiert wird.

2. Gegenstand nach Patentanspruch 1 **dadurch gekennzeichnet, dass** die Vorrichtung ein grobporiger Gesichtshandschuh darstellt.

3. Gegenstand nach einem der vorangehenden Ansprüche **dadurch gekennzeichnet, dass** die Zubereitung als W/O- Creme vorliegt.

4. Gegenstand nach einem der vorangehenden Ansprüche **dadurch gekennzeichnet, dass** ein Wirkstoff enthalten ist.

5. Gegenstand nach einem der vorangehenden Ansprüche **dadurch gekennzeichnet, dass** der Wirkstoff aus der Gruppe Antioxidantien gewählt wird.

6. Gegenstand nach einem der vorangehenden Ansprüche **dadurch gekennzeichnet, dass** der Wirkstoff Ubichinon und/oder Ubichinol darstellt.

7. Gegenstand nach einem der vorangehenden Ansprüche **dadurch gekennzeichnet, dass** der Wirkstoff Kreatin und/oder Kreatinin darstellt.

8. Gegenstand nach einem der vorangehenden Ansprüche **dadurch gekennzeichnet, dass** die Dosierung der Zubereitung durch die Vorrichtung bei ihrer Anwendung erfolgt.

9. Verwendung einer Vorrichtung nach einem der vorangehenden Ansprüche zur Erhöhung der Penetration eines innerhalb von 30 s angewendeten kosmetischen und/oder dermatologischen Wirkstoffes.

10. Verwendung eines Gegenstands nach einem der vorangehenden Ansprüche zur Verbesserung des Energiestoffwechsels.

11. Verwendung eines Gegenstands nach einem der vorangehenden Ansprüche zur Anregung der Kollagenneogenese.

12. Nicht therapeutisches Verfahren zur Behandlung und Prophylaxe degenerativer Hauterscheinungen durch Anwenden eines Gegenstandes nach einem der vorangegangenen Ansprüche.
